Europäisches Patentamt

European Patent Office

Office européen des brevets

(19)

(11) Veröffentlichungsnummer: **0 120 991**
**B1**

(12) **EUROPÄISCHE PATENTSCHRIFT**

(45) Veröffentlichungstag der Patentschrift: **14.03.90**

(21) Anmeldenummer: **83111628.0**

(22) Anmeldetag: **22.11.83**

(51) Int. Cl.⁵: **G 08 B 21/00,** G 08 B 23/00, G 01 W 1/00

(54) Verfahren zur Vorwarnung von Patienten mit klimatisch beeinflussten Krankheiten, wie z.B. Epilepsie und Herzinfarkt, und Schaltungsanordnung hierzu.

(30) Priorität: **25.03.83 DE 3310908**

(43) Veröffentlichungstag der Anmeldung:
**10.10.84 Patentblatt 84/41**

(45) Bekanntmachung des Hinweises auf die Patenterteilung:
**14.03.90 Patentblatt 90/11**

(84) Benannte Vertragsstaaten:
**AT BE CH DE FR GB IT LI LU NL SE**

(56) Entgegenhaltungen:
**CH-A- 456 978**
**CH-A- 493 856**
**US-A-3 753 116**
**US-A-3 810 137**

**ARCHIV FÜR METEOROLOGIE, GEOPHYSIK UND BIOKLIMATOLOGIE, Serie A, Meteorologie und Geophysik, Vol. 29, No. 1-2, 1980 Wien-New York H. BAUMER, J EICHMEIER "Eine Anlage zur Registrierung der Atmospherics bei 10 und 27 kHz" Seiten 143-155**

(73) Patentinhaber: **ATMOSPHERIC WEATHER ANALYSIS SYSTEMS, AWAS (GmbH & Co.) KG**
**Amsinckstrasse 39**
**D-2000 Hamburg 1 (DE)**

(72) Erfinder: **Ruhenstroth-Bauer, Gerhard, Prof. Dr.**
**Spietzelbergerstrasse 11**
**D-8032 Gräfelfing (DE)**

(74) Vertreter: **Dreiss, Hosenthien & Fuhlendorf**
**Gerokstrasse 6**
**D-7000 Stuttgart 1 (DE)**

(56) Entgegenhaltungen:
**ARCHIV FÜR METEOROLOGIE, GEOPHYSIK UND BIOKLIMATOLOGIE, Serie B, Climatology, Environmetal Meteorology, Radiation Research, Vol. 20, No. 1-2, 1981, Wien-New York W. SÖNNING, H. BAUMER, J. EICHMEIER "Die Atmospherics-Aktivität bei 10 und 27 kHz als Indikator für die Dynamik der troposphärischen Wettervorgänge" Seiten 299-312**

**Technischer Informationsdienst Bundesverband Druck e.V., Abt. Technik und Forschung, Fachbereich Tiefdruck, Veröffentlichung II/1982, H. Baumer; "Die Meteorotropie eines Dichromat-Gelatinesystems"**

EP 0 120 991 B1

**Beschreibung**

Die Erfindung betrifft ein Verfahren zur Vorwarnung von Patienten mit klimatisch beeinflußten Krankheiten, wie z.B. Epilepsie und Herzinfarkt, sowie eine Schaltungsanordnung hierzu.

Man hat gefühlsmäßig schon seit alters her erkannt, daß das Wetter ein sehr wichtiger Faktor beim Auftreten bestimmter Krankheiten ist. Hierzu gehören insbesondere die Epilepsie und der Herzinfarkt. Es wurde schon vielfältig versucht, die sogenannten klassischen Wetterparameter, wie die absolute oder relative Feuchte, die mittlere Temperatur, bestimmte Wetterphasen, usw. mit den Anfallshäufigkeiten der genannten Krankheiten zu korrelieren, insbesondere um gegebenenfalls bestimmte prophylaktische Maßnahmen treffen zu können. Man hat hierzu bis jetzt nur wenig aussagekräftige Messungen machen können.

Der Erfindung liegt die Aufgabe zugrunde, ein Verfahren zu schaffen, das die Vorhersage klimatisch ungünstiger bzw. günstiger Bedingungen für klimatisch beeinflußte Krankheiten, insbesondere Anfallskrankheiten wie Epilepsie und Herzinfarkt, ermöglicht. Außerdem soll eine Schaltungsanordnung zur Vorwarnung geschaffen werden.

Erfindungsgemäß wird diese Aufgabe durch ein Verfahren gelöst, das dadurch gekennzeichnet ist, daß sie sinoidalen Sphärik-Impulse der Frequenz 28 kHz und die sinoidalen Sphärik-Impulse der Frequenz 10 kHz mittels eines entsprechende Antennen, Verstärker und Filter aufweisenden Sphärik-Selektors gemessen werden, daß die Impulsrate dieser Sphärik-Impulse über einen vorbestimmten Zeitraum ermittelt, daß die Differenz der Impulsraten der Sphärik-Impulse von 28 kHz und der von 10 kHz gebildet wird, und daß bei Überwiegen der Sphärik-Impulse von 28 kHz und bei Feststellung der Abwesenheit von Sphärikimpulsen von 50 kHz Kommunikationseinrichtungen zur Vorwarnung von Patienten betätigt werden.

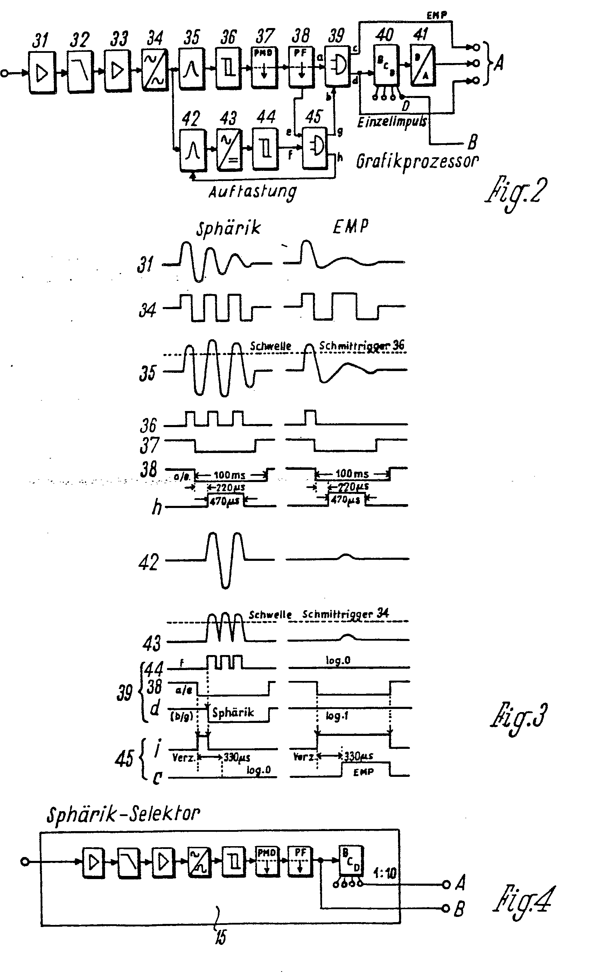

Die Erfindung geht also von der Erkenntnis aus, daß für die Klimaeinflüsse auf die genannten Krankheiten nicht das Wetter selbst, sondern bestimmte bei der Wetterbildung auftretende elektromagnetische Schwingungsphänomene, nämlich sog. "Sphärikimpulse" (spherics) bestimmter Frequenz und in bestimmter Kombination, verantwortlich sind. Bei den Sphärik-Impulsen handelt es sich um eine gedämpfte Schwingung mit fünf bis sechs Halbwellen und weitgehend exakter Sinusform, die offensichtlich bei Entladungsvorgängen in der Atmosphäre gebildet werden (siehe die Darstellung eines Sphärik-Impulses in der ersten Zeile, linke Spalte, in Fig. 3). Die Form derartiger Sphärik-Impulse ist bekannt, vgl. *Eichmeier, J.,* Eine Anlage zur Registrierung der Atmospherics bei 10 und 27 kHz. Arch. Meteor. Geophysik, Bioklimat. A, 29: 143—155 (1980) und *Sönning, W., Baumer, H. und Eichmeier, J.,* Die Atmospherics-Aktivität bei 10 und 27 kHz als Indikator für die Dynamik der troposphärischen Wettervorgänge, Arch. Meteor. Geophysik, Bioklimat. B, 29, 299—312 (1981).

Man ist bei der Analyse der Wetterabhängigkeit eines im Tiefdruck verwendeten Dichromat- Gelatinesystems bereits auf die biologische Wirkung bestimmter Sphärik-Impulse gestoßen. Die hohe Korrelation des Auftritts dieser Sphärik-Impulse, d.h. der angegebenen Kombination derselben, mit den angegebenen Krankheitsbildern, die im folgenden insbesondere für die Epilepsie und für Herzinfarkte nachgewiesen werden, bringt überdies weiterhin die Erkenntnis, daß auf diese Art und Weise gefährdete Patienten vorgewarnt werden können, wenn gleichzeitig die Abwesenheit von Sphärikimpulse von 50 kHz festgestellt wird.

An sich wird eine Vorwarnung von Patienten in Artikel von H. Baumer: "Die Meteorotropie eines Dichromat-Gelatinsystems" in der Veröffentlichung II/1982, Technischer Informationsdienst, Abteilung Technik und Forschung, Fachbereich Tiefdruck, des Bundesverbandes Druck e.V., Postfach 106 g, Weinbergstrape 2, 6200 Wresbaden 1, erwähnt.

Ausführungsbeispiele der Erfindung werden im folgenden unter Bezugnahme auf die beigefügten Zeichnungen näher beschrieben. Es stellen dar:

Figur 1 den Eingangsteil einer erfindungsgemäßen Schaltungsanordnung;
Figur 2 die detailliertere Darstellung der in Fig. 1 verwendeten Sphärik-Selektoren;
Figur 3 verschiedene Impulsverläufe an verschiedenen Stellen der Schaltung nach Fig. 2;
Figur 4 die detailliertere Darstellung eines weiteren der in Fig. 1 verwendeten Sphärik-Selektoren;
Figur 5 einen Ausgabe-Prozessor, wie er im Zusammenhang mit der Schaltung nach Fig. 1 Verwendung findet;
Figur 6 ein Schaubild zur Korrelation des Auftretens bestimmter Kombinationen von Sphärik-Impulsen mit der Häufigkeit von Epilepsie-Anfällen bzw. der Häufigkeit von Herzinfarkten an bestimmten Probandengruppen.

Der Empfang der Sphärik-Impulse mit 28 kHz (genau: 28018,17 Hz) erfolgt mit Hilfe von vier horizontal angeordneten Ferritantennen 1 bis 4. Die vier Ferritantennen sind horizontal in Richtung der vier Himmelsrichtungen ausgerichtet. Die horizontale Anordnung entspricht der Tatsache, daß es sich bei diesen Sphärik-Impulsen um elektromagnetische Wellen mit horizontal polarisierter Magnetfeld- Komponente handelt. Ferner ist eine vertikal angeordnete Breitbandantrene 5 vorgesehen. Sie dient zur Aufnahme der Sphärik-Impulse mit vertikal polarisierter elektrischer Feldkomponente und zur Ableitung von Sphärik-Impulsen mit Frequenzen zwischen 3—100 kHz. Die Messungen haben ergeben, daß in diesem Bereich spektrale Maxima von Sphärik-Impulsen existieren. Sie sind mit a, b, c, und d bezeichnet. Im vorliegenden Zusammenhang sind die Sphärik-Impulse mit einer Frequenz von 10,38 kHz (im folgenden vereinfacht: 10 kHz) von besonderer Bedeutung.

Die Antennen 1 bis 4 sind über Vorverstärker 6 bis 9 mit den Sphärik-Selektoren 10 bis 13 verbunden. Sowohl die Antennen 1 bis 4 wie auch die Sphärik-Selektoren 10 bis 13 für die Sphärik-Impulse mit 28 kHz sind entsprechend den Himmelsrichtungen, aus denen die genannten Antennen die Sphärik-Impulse empfangen, mit $28_I$, $28_{II}$, $28_{III}$, $28_{IV}$ bezeichnet.

Die Breitbandantenne 5 ist über den Vorverstärker 14 mit den Sphärik-Selektoren 15 bis 20 für die genannten Frequenzen verbunden.

Zur Reichweitenregelung dient die Regelung ver Vorverstärker 6 bis 9, 14, die das von einem Kontrollsender mit stets konstanter Sendeamplitude abgestrahlte Kontrollsignal empfängt und je nach Höhe dieses Empfangs die genannten Vorverstärker steuert, so daß unterschiedliche Dämpfungen in der Übertragungsstrecke von der Stelle des Entstehens der Sphärik-Impulse bis zur Stelle der Anordnung der Antennen 1 bis 5 ausgeglichen werden. Die Einstellung erfolgt über die Steuerschaltung 22.

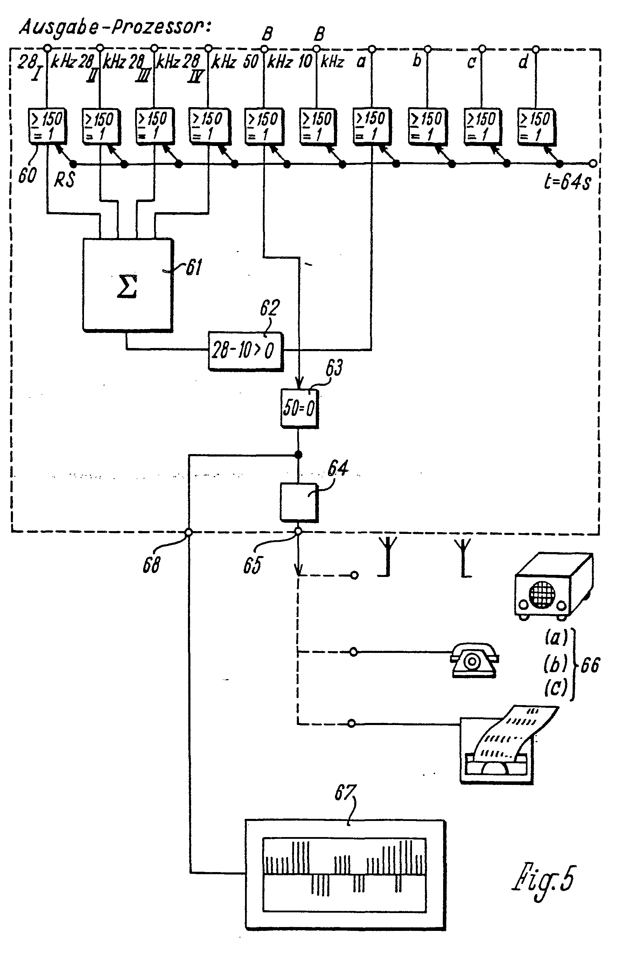

Jeder der Sphärik-Selektoren 10 bis 13, 15 bis 20 ist mit zwei Ausgängen versehen. Die oberen Ausgänge A führen an nicht näher beziechnete Schreiber; die unteren Ausgänge B führen an die Eingänge der Schaltungen nach Fig. 5.

Die Sphärik-Selektoren 10 bis 13 haben den in Fig. 2 gezeigten Aufbau. Er dient dazu, einen Sphärik-Impuls, wie er auf der linken Seite der ersten Zeile in Fig. 3 dargestellt ist, zu identifizieren und gleichzeitig Impulse der auf der rechten Seite der Zeile 1 in Fig. 2 gezeigten Art, sog. EMP-Impulse, bei der Auswertung auszuscheiden. Bei einem derartigen "Electro-Magnetic-Pulse" (=EMP) handelt es sich um den im Nahbereich entstehenden "Urimpuls", der biologisch unwirksam ist und bei der Messung ausgeschieden werden muß. Als Urimpuls kann er deshalb bezeichnet werden, da in dieser Form sämtliche Frequenzen bis in den MHz-Bereich hinein vorhanden sind und einiges für die Hypothese spricht, daß aus ihm durch Filterung an durch bestimmte meteorotrophe Eigenschaften gekennzeichnete Luftmassen die biologisch wirksamen Sphärik-Impulse entstehen.

Der Sphärik-Selektor 10 wird gebildet durch einen Eingangsverstärker 31, ein Tiefpaßfilter 32, einen weiteren Verstärker 33, der jeweils auf die spezielle Frequenz des betreffenden Sphärik-Selektors abgestimmt ist, einen Begrenzer 34, ein Bandpaßfilter 35, einen Schmitt-Trigger 36, einen Pulse-Missing-Detektor 37, einen Pulse-Former 38, ein Logik-Netzwerk 39 und einen Deizmalzähler 40. Am Ausgang des Begrenzers 34 liegt ferner, parallel zu den genannten Schaltungseinheiten, an: ein weiterer Bandpaßfilter 42 mit verengtem Durchlaßbereich, ein Doppelweg-Gleichrichter 43, ein weiterer Schmitt-Trigger 44, sowie ein zweites Logik-Netzwerk 45. Die Eingänge des Logik-Netzwerks 39 sind mit a und b, die des zweiten Logik-Netzwerks 45 mit e und f bezeichnet; die Ausgänge des Logik-Netzwerks 39 sind mit c und d, die Ausgänge des Logik-Netzwerks 45 mit g und h bezeichnet.

Die Ausgänge der bezeichneten Schalteinheiten sind in Fig. 3 teilweise in den entsprechend bezeichneten Zeilen dargestellt, und zwar in der linken Spalte für den Fall eines Sphärik-Impulses am Eingang und in der rechten Spalte für den Fall eines EMP-Impulses am Eingang. Die Schaltung führt dazu, daß am Ausgang d des Logik-Netzwerks 39 im Falle eines Sphärik-Impulses ein Signal erscheint, im Falle eines EMP-Impulses jedoch nicht. Beim Auftreten eines EMP-Impulses tritt am Ausgang d des Logik-Netzwerks 39 kein Impuls auf. Der EMP-Impuls kann am Ausgang c abgenommen werden. Zur Funktionsweise im einzelnen:

Das in den Verstärkern 31 und 33 verstärkte und im Tiefpaßfilter 32 von höherfrequenten Komponeten befreite Signal wird im Begrenzer 34 auf eine einheitliche Amplitude gebracht. Durch das Bandpaßfilter 5 und den Schmitt-Trigger 6 wird der Durchlaßbereich festgelegt (z.B. 20 bis 30 kHz). Gleichzeitig erhält das Signal Rechteckverlauf (siehe den entsprechenden Kurvenverlauf am Ausgang des Schmitt-Triggers 36 in Fig. 3). Bei dem Pulse-Missing-Detektor 37 handelt es sich im Prinzip um einen nachtriggerbaren Monovibrator, d.h. eine Schaltungseinheit, die von einer negativen Flanke eines Impulses umgeschaltet wird und nach einer bestimmten einstellbaren Zeit wieder in ihren Ausgangszustand zurückfällt, sofern nicht erneut ein Impuls anliegt. Das heißt: Der Puls-Missing-Detektor 37 wird von der nacheilenden Flanke der ersten Halbwelle des Sphärik-Impulses von "1" auf "0" umgeschaltet. Er fällt aber erste dann wieder auf "1" zurück und kann damit ein weiteres Signal zur weiteren Impulsbildung an den nachgeschalteten Pulseformer 38 abgeben, wenn über eine bestimmte Zeitspanne, innerhalb der er auf "1" zurückgegangen sein muß, keine weitere Halbwelle anliegt. Auf diese Weise werden technische Dauerschwingungen, wie sie etwa von elektrischen Geräten in der Umgebung der Antennen erzeugt werden (sog. technische Schwingungen) ausgeschaltet. In dem Pulseformer 38 erfolgt dann die Ausbildung eines bis 100 ms langen Impulses. Dieser Impuls geht auch an das Logiknetzwerk 45, das daraus nach einer bestimmten voreingestellten Verzögerung am Ausgang h einen Auftastimpuls ableitet, der den Schaltweg über die Schaltungseinheiten 42, 43, 44 öffnet. Damit kann der Sphärik-Impuls vom EMP-Impuls unterschieden werden. Beim Sphärik-Impuls folgen auf die erste Halbwelle noch weitere Halbwellen mit einer Amplitude, die zwar geringer als die der ersten Halbwelle ist, die aber denoch hoch genug ist, um den nachgeschalteten Schmitt-Trigger 44 zu schalten; beim EMP-Impuls ist dies nicht der Fall. Am Ausgang f entsteht somit nur dann ein Signal, wenn es sich am Eingang um einen sinoidalen Sphärik-Impuls gehandelt hat. Wenn also sowohl auf der Leitung a) am Ausgang des Pulseformers 38 (Monovibrator mit bis 100 ms Pulsebreite), als auch auf der Leitung g am Ausgang des zweiten Logiknetzwerks 45 gleichzeitig Signale anliegen, d.h. wenn am Eingang ein Sphärik-Impuls vorgelegen hat, dann erscheint am Ausgang d

3

des ersten Logiknetzwerks 39 ein Impuls; vgl. dazu die drittletzte Zeile, linke Spalte in Fig. 3. Am Ausgang des Dezimalzählers 40 erfolgt eine Herabsetzung der Impulsrate um 1:10.

Figure 4 zeigt als Beispiel für einen der Sphärik-Selektoren 15 bis 20 den Sphärik-Selektor 15. Er ist im Prinzip gleich aufgebaut wie der Sphärik-Selektor nach Fig. 2, jedoch mit dem Unterschied, daß die Schaltungseinheiten 42 bis 45 nicht vorgesehen sind. Auf sie kann im Falle dieser Frequenzen verzichtet werden, da es sich dabei um vertikal polarisierte magnetische Felder handelt, die keinen EMP-Anteil aufweisen, der ausgeschieden werden müßte.

Die jeweils 2 Ausgänge der Sphärik-Selektoren sind mit A und B zeichnet. Die Ausgänge A gelangen an einen nicht näher darzustellenden Scheiber, die Ausgänge B an den Ausgabe-Prozessor nach Fig. 5. Die Kanäle sind dabei mit den entsprechenden Frequenzen bezeichnet. Die Impulsfolgefrequenzen werden z.B. in einem Abtastzyklus von 8 Minuten bei einer Impulsrate von 0 bis 320 Hz (Klasse 1), 320 bis 2560 Hz (Klasse 2), und bei mehr als 2560 Hz (Klasse 3) ausgegeben.

Der für die erfindungsgemäße Vorwarnung von Patienten mit klimatisch beeinflußten Krankheiten maßgebliche Ausgabe-Prozessor ist in Fig. 5 dargestellt. Die Eingänge B der betreffenden Kanäle gelangen zunächst auf Zähler 60, die für je 150 Signale am Eingang ein Ausgangssignal abgeben und jeweils nach 64 s zurückgeschaltet werden. Ist innerhalb dieser Taktzeit von 64 s ein Ausgangssignal an einem Zähler 60 erschienen, so war die Häufigkeit der im vorgeschalteten Sphärik-Selektor festgestellten Sphärik-Impulse größer als 150/64, also größer als ungefähr 2,5 Hz. Die Ausgangssignale der Zähler 60 der Kanäle $28_I$, $28_{II}$, $28_{III}$, $28_{IV}$ kHz werden in der Summenschaltung 61 addiert. In dem Subtrahier-Netzwerk 62 wird hiervon die im Zeittakt angefallene Zahl der Impulse mit 10 kHz subtrahiert und die Differenz, sofern $\neq 0$, am Ausgang des Subtrahier-Netzwerks 82 abgegeben. Von dort gelangt sie an ein Netzwerk 63, das dann einen Impuls weitergibt, wenn nicht gleichzeitig auch Sphärik-Impulse im Bereich 50 kHz gemessen werden. Dies beruht darauf, daß Sphärik-Impulse dieser Frequenz möglicherweise als Störer zu werten sind, die die biologische Wirksamkeit der Sphärik-Impulse von 28 kHz −10 kHz beeinträchtigen. Mit 64 ist ein Kodierer bezeichnet, der die Ausgangssignale des Netzwerks 63 in für die nachgeschalteten Kommunikations-Einrichtungen 66 geeignete Form übersetzt Als geeignete Kommunikationseinrichtung sind drei Beispiele dargestellt, nämlich einmal die Rundfunkübertragung, mit (a) bezeichnet, die Telefonübertragung, mit (b) bezeichnet, sowie die Fernschreibübertragung, mit (c) bezeichnet. Eine vierte Möglichkeit wäre der Empfang durch eine besondere Fernseh-Gemeinschaftsantenne, an die ein Teilnehmer über Kabel angeschlossen ist.

Die Übertragung einer Vorwarnung auf einem der dargestellten Wege erfolgt also in folgenden Fällen:
—mit einer Frequenz von mehr als 1,0 Hz treten in den dem Frequenzband von 28 kHz zugeordneten Kanälen Sphärik-Impulse auf; es handelt sich dabei um Impulse, wie sie in der linken Spalte von Fig. 3 in der ersten Zeile wiedergegeben sind.
—Die Häufigkeit der Sphärik-Impulse mit mehr als 1,0 Hz ist größer als die derjenigen mit 10 kHz;
—es treten nicht gleichzeitig Sphärik-Impulse im Frequenzband 50 kHz auf; dabei werden durch die betreffende Schaltung
—sog. EMP-Impulse der in der ersten Zeile, rechte Spalte, in Fig. 3 dargestellten Form ausgeschaltet; ferner werden
—technische Dauerschwingungen ausgeschaltet.

Es hat sich nun gezeigt, daß die Signale, wie sie auf diese Weise abgeleitet werden und am Ausgang 65 des Ausgabe-Prozessors nach Fig. 5 zur Verfügung stehen, mit bestimmten klimatisch beeinflußten Krankheiten, als deren Beispiele im folgenden Epilepsie und Herzinfarkt näher erläutert werden, korrelieren, so daß die dargestellte Schaltung eine geeignete Vorwarn-Einrichtung für Patienten, die an diesen Krankheiten leiden, darstellt. Bei Auftreten einer derartigen Vorwarnung können die Patienten vorbeugende Medikamente einnehmen bzw. bestimmte Risikosituationen vermeiden.

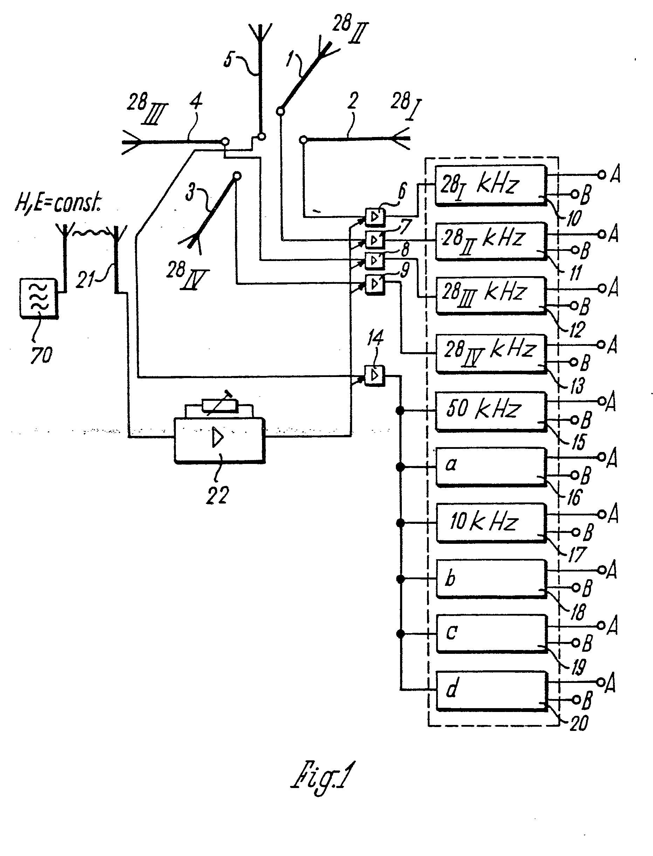

Zur Schaltung nach Fig. 1 bis 5 ist ergänzend zu bemerken, daß die dargestellten Kanäle für die Frequenzen zwischen 3 und 100 kHz deshalb in die Darstellung mit einbezogen wurden, weil auch bei diesen Frequenzen, wie erwähnt, Sphärik-Impulse auftreten. Ob und inwieweit sie biologische Wirksamkeit entfalten, ist noch nicht endgültig geklärt.

Beim Ausgabe-Prozessor nach Fig. 5 ist noch ein Ausgang 68 eingezeichnet, über den ein Schreiber 67 die Differenz der Impulsrate der 28 kHz- und der 10 kHz-Sphärik-Impulse schreibt.

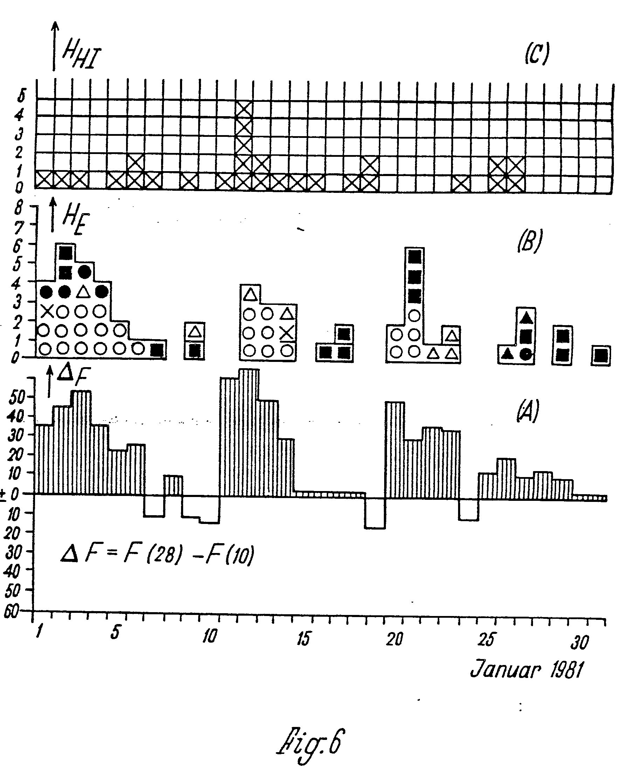

Fig. 6 zeigt in der Kurve (A) das Ergebnis eines solchen Aufschriebs für den Zeitraum Januar 1981. Solange die Kurve oberhalb der Null-Linie verläuft, bedeutet dies, daß die Sphärik-Impulse mit 28 kHz überwogen haben; sofern die Kurve unterhalb der Null-Linie verläuft, überwogen die Sphärik-Impulse mit 10 kHz.

Dementsprechend wurde in der Kurve (B) die Häufigkeit $H_E$ von Epilepsie-Anfällen von sechs Patienten aufgetragen (die unterschiedlichen Symbole in (B) bezeichnen die verschiedenen Patienten). Die Kurve (C) zeigt während desselben Zeitraums die Häifigkeit von Herzinfarkten in insgesamt vier Münchener Kliniken.

Schon die grafische Darstellung nach Fig. 6 zeigt eine hohe Korrelation zwischen einem hohen Wert der Differenz von 28 kHz- und 10 kHz-Spheriks-Impulsen und der Häufigkeit von epileptischen Anfällen und Herzinfarkten. Ergänzend sind hierzu noch die folgenden Angaben zu machen:

Für die sechs Probanden, für die die Häufigkeit der epileptischen Anfälle im betrachteten Zeitraum untersucht und mit der Häufigkeit des Auftretens von Sphärik-Impulsen in der dargestellten Weise korreliert worden ist, ergaben sich die folgenden Spearman'schen Rangkorrelationskoeffizienten (zur Definition von z.B. Hartung/Elpelt/Kösener, Statistik, R. Oldenbourg Verlag, München/Wien, 1982, S. 79):

4

### Spearman'sche Rangkorrelationskoeffizienten

| Proband | Zahl der Anfälle | 28 kHz-Sphärik-Impuls | 10 kHz-Sphärik-Impuls |
|---------|------------------|----------------------|----------------------|
| A | 94 | 0.18 | −0.13 |
| B | 10 | 0.11 | −0.11 |
| C | 62 | 0.10 | 0.06 |
| D | 31 | 0.28 | −0.15 |
| E | 71 | 0.20 | −0.24 |
| F | 47 | −0.07 | 0.03 |

Aus der positiven Korrelation mit den 28 kHz-Sphärik-Impulsen und der negativen Korrelation mit den 10 kHz-Sphärik-Impulsen folgt, daß die Differenz beider offensichtlich die entscheidende Bedingungen darstellt. Die in der vorstehenden Tabelle angegebenen Zahlen beziehen sich jedoch nicht nur auf einen Monat, wie Fig. 6, sondern auf einen Zeitraum von 7 Beobachtungsmonaten. Die Tatsache, daß die Korrelationen bei den einzelnen Probanden unterschiedlich sind, ist im übrigen ein Hinweis auf die Beobachtung, daß es bei der Epilepsie Untergruppen gibt, die offenbar auch durch die klimatischen Bedingungen unterschiedlich beeinflußt werden.

Um die Korrelation eines positiven Wertes für die Differenz der 28 kHz-Sphärik-Impulse und der 10 kHz-Sphärik-Impulse mit dem Auftreten von Herzinfarkten weiter zu untersuchen, wurde der Herzinfarkt-Kalender von vier Münchener Kliniken in der Zeit vom 1.1.1981 bis zum 31.7.1981 insgesamt ausgewertet. Dabei ergaben sich die folgenden Spearman'schen Rangkorrelationskoeffizienten (Angabe der Irrtumswahrscheinlichkeit in Klammern):

| 10 kHz-Sphärik-Impuls | −0.10 | (0.15) |
|------------------------|-------|--------|
| 28 kHz-Sphärik-Impuls | 0.13 | (0.065) |
| absolute Feuchte | −0.17 | (0.015) |
| mittlere Temperatur | −0.13 | (0.056) |
| Höchsttemperatur | −0.12 | (0.072) |
| Tiefsttemperatur | −0.15 | (0.028) |
| Wetterphase | 0.02 | (0.78) |
| Wettervorgang | −0.05 | (0.47) |

In dieser Tabelle sind außer den genannten Sphärik-Impulsen noch weitere Wetterparameter aufgelistet, nämlich die absolute Feuchte, die mittlere Temperatur, die Höchsttemperatur, die Tiefsttemperatur, die Wetterphase (Übergang von schlechtem Wetter zu gutem Wetter bzw. umgekehrt) und der Wettervorgang (z.B. Regen). Die Tabelle zeigt, daß die üblichen Wetterparameter, deren Beobachtung zunächst bei der Untersuchung der Wetterempfindlichkeit naheliegt, mit dem Auftreten der Herzinfarkte nicht positiv korrelieren, während die erfindungsgemäß gefundenen 28 kHz Sphärik-Impulse dies tun.

### Patentansprüche

1. Verfahren zur Vorwarnung von Patienten mit klimatisch beeinflußten Krankheiten, wie z.B. Epilepsie und Herzinfarkt, vor anfallsbegünstigenden Bedingungen, dadurch gekennzeichnet, daß die sinoidalen Sphärik-Impulse der Frequenz 28 kHz und die sinoidalen Sphärik-Impulse der Frequenz 10 kHz mittels eines entsprechende Antennen (1 bis 5), Verstärker (6 bis 9, 14) und Filter (35, 36) aufweisenden Sphärik-Selektors (10 bis 13, 17) gemessen werden, daß die Impulsrate dieser Sphärik-Impulse über einen vorbestimmten Zeitraum ermittelt, daß die Differenz der Impulsraten der Sphärik-Impulse von 28 kHz und der von 10 kHz gebildet wird, und daß bei Überwiegen der Sphärik-Impulse von 28 kHz und bei Feststellung der Abwesenheit von Sphärikimpulsen von 50 kHz Kommunikationseinrichtungen (66) zur Vorwarnung von Patienten betätigt werden.

2. Verfahren nach Anspruch 1, dadurch gekennzeichnet, daß bei den Freqeunzen 28 kHz und 10 kHz nur Impulsraten von mehr als 1,0 Hz berücksichtigt werden.

3. Schaltungsanordnung zur Vorwarnung von Patienten mit klimatisch beeinflußten Krankheiten, wie z.B. Epilepsie und Herzinfarkt, vor anfallsbegünstigenden Bedingungen, dadurch gekennzeichnet, daß zum Empfang von Sphärik-Impulsen mit horizontal polarisierter Magnetfeld-Komponente zumindest eine vertikal angeordnete Ferritantenne und daß zum Empfang von Sphärik-Impulsen mit einer vertikal polarisierten Magnetfeldkomponente zumindest eine horizontal angeordnete weitere Antenne (5) vorgesehen ist, daß ferner den Antennen Sphärik-Selektoren (10 bis 13, 17) nachgeschaltet sind, die Filter (35, 36) für die betreffenden Frequenzen sowie Schaltungseinrichtungen (37), die bei technischen Dauerschwingungen eine Sperrung bewirken, sowie—bei den Sphärik-Selektoren (10 bis 13) für die Sphärik-Impulse mit vertikal polarisiertem magnetischem Feld—weitere logische Schaltungseinrichtungen (38, 39, 45) zur Sperrung beim Auftreten von EMP-Impulsen aufweisen, und daß ferner weitere logische Schaltungseinheiten (60) dann die Impulsraten der betreffenden Sphärik-Impulse an ein Subtrahier-Netzwerk (62) weiterleiten, wenn diese einen vorbestimmten Wert überschreiten, und daß ferner in dem Subtrahier-Netzwerk die Impulsraten der Sphärik-Impulse mit 10 kHz von denen der Sphärik-Impulse mit 28 kHz abgezogen werden, und daß das Ausgangssignal dieses Subtrahier-Netzwerks (62) das Vorwarnsignal darstellt, und daß ein weiteres Netzwerk (63) vorgesehen ist, an das das Signal vom Ausgang des genannten Subtrahier-Netzwerks (62) gelangt, und an das ferner die Sphärik-Impulse mit einer Frequenz von 50 kHz gelangen, und das nur dann die Ausgangssignale von dem erstgenannten Subtrahier-Netzwerk (62) weiterleitet, wenn keine Sphärik-Impulse der Frequenz 50 kHz vorliegen.

4. Schaltungsanordnung nach Anspruch 3, dadurch gekennzeichnet, daß die Häufigkeit des Auftretens der Sphärik-Impulse, bei deren Überschreiten die genannten Schaltungseinrichtungen (60) die Anzahl der von den Sphärik-Selektoren gemessenen Sphärik-Impulse pro Zeiteinheit an das Subtrahier-Netzwerk weitergeben, 1,0 Hz ist.

5. Schaltungsanordnung nach Anspruch 3, dadurch gekennzeichnet, daß zur Begrenzung der Reichweite der Antennen die Steuerung der den Sphärik-Selektoren (10 bis 13, 15 bis 20) vorgeschalteten Vorverstärker mittels eines Steuersignals erfolgt, das von der Stärker des Empfangssignals abhängt, das mittels einer weiteren Antenne (21) von einem mit konstanter Feldstärker sendenden Kontrollsender empfangen wird.

**Revendications**

1. Procédé pour alerter des patients atteints de maladies influencées par le climat, comme par exemple l'épilepsie et l'infarctus du myocarde, avant l'apparition de conditions pouvant favoriser une crise, caractérisé en ce que les impulsions atmosphériques sinusoïdales d'une fréquence de 28 kHz et les impulsions atmosphériques sinusoïdales d'une fréquence de 10 kHz sont mesurées au moyen d'un sélecteur atmosphérique (10 à 13, 17) muni d'antennes (1 à 5), d'amplificateurs (6 à 9, 14) et de filtres (35, 36) appropriès, que le taux d'impulsion de ces impulsions atmosphériques est evalue sur une période prédéterminée, que la différence entre les taux d'impulsion des impulsions atmosphériques de 28 kHz et de celles de 10 kHz est formée, et que lors du depassement des impulsions atmosphériques de 28 kHz et lors de la constatation de l'absence d'impulsions atmosphériques de 50 kHz, des dispositifs de communication (66) sont mis en oeuvre pour alerter le patient.

2. Procédé selon la revendication 1, caractérisé en ce que pour les fréquences de 28 kHz et de 10 kHz, seuls des taux d'impulsion supérieurs à 1,0 Hz sont pris en considération.

3. Dispositif de circuit pour alerter des patients atteints de maladiès influencées par le climat, comme par exemple l'epilepsie et l'infarctus du myocarde, avant l'apparition de conditions favorisant une crise, caractérisé en ce que pour capter les impulsions atmosphériques avec des composants de champ magnétique à polarisation horizontale, il est prevu au moins une antenne de ferrite disposée verticalement, et que pour capter les impulsions atmosphériques avec des composants de champ magnétique à polarisation verticale, il est prévu au moins une antenne supplémentaire (5) disposée horizontalement, que de plus, des selecteurs atmosphériques (10 à 13, 17) sont montés derrière les antennes, comportant des filtres (35, 36) pour les fréquences concernées ainsi que des dispositifs de commutation (37) qui, en cas d'oscillations permanentes techniques, effectuent un blocage, ainsi que—dans le cas des sélecteurs atmosphériques (10 à 13) pour les impulsions atmosphériques avec un champ magnétique à polarisation verticale—d'autres dispositifs de circuits logiques (38, 39, 45) pour effectuer le blocage lors de l'apparition d'impulsions produites par une explosion atomique (EMP), et que d'autres unites de commutation logiques (60) transmettent ensuite les taux d'impulsion des impulsions atmosphériques concernées à un réseau de soustraction (62), lorsque ceux-ci dépassent une valeur prédéterminée, et qu'en outre, dans le réseau de soustraction, les taux d'impulsion des impulsions atmosphériques de 10 kHz sont retirees de celles des impulsions atmosphériques de 28 kHz, et que le signal de sortie de ce réseau de soustraction (62) représente le signal d'alerte, et qu'un autre réseau (63) est prévu, auquel parvient le signal de sortie du réseau de soustraction (62) mentionné, et auquel parviennent en outre les impulsions atmosphériques de 50 kHz, et qui ne transmet le signal de sortie du réseau de soustraction (62) mentionné en premier que lorsqu'il n'existe pas d'impulsion atmosphérique d'une fréquence de 50 kHz.

4. Dispositif de commutation selon la revendication 3, caractérisé en ce que la fréquence d'apparition des impulsions atmosphériques, pour le dépassement desquelles les dispositifs de commutation (60) mentionnés transmettent vers le réseau de soustraction le nombre des impulsions atmosphériques par unité de temps mesuré par le sélecteur atmosphérique est de 1.0 Hz.

5. Dispositif de commutation selon la revendication 3, caractérisé en ce que pour limiter le rayon d'action des antennes, la commande des préamplificateurs montés en amont des selecteurs atmosphériques (10 à 13, 15 à 20) s'effectue au moyen d'un signal de commande, qui est fonction de l'intensité du signal capté, qui est capté au moyen d'une autre antenne (21) d'un émetteur de contrôlé émettant avec une intensité de champ constante.

**Claims**

1. Process for pre-warning patients having climatically influenced diseases such as epilepsy and cardiac infarction of conditions promoting an attack, characterised in that the sinoidal spherics pulses of the frequency 28 kHz and the sinoidal spherics pulses of the frequency 10 kHz are measured by means of a spherics selector (10 to 13, 17) comprising appropriate antennas (1 to 5), amplifiers (6 to 9, 14) and filters (35, 36), in that the pulse rate of said spherics pulses is measured over a pre-determined period, in that the difference of the pulse rates of the 28 kHz spherics pulses and those of the 10 kHz spherics pulses is determined, and in that communication means (66) are actuated to pre-warn the patient, if the 28 kHz spherics pulses prevail and the absence of 50 kHz spherics pulses is established.

2. Process according to Claim 1, characterised in that in the case of the 28 kHz and 10 kHz frequencies only pulse rates of more than 1,0 Hz are considered.

3. Circuit arrangement for pre-warning patients having climatically influenced diseases such as epilepsy and cardiac infarction of conditions promoting an attack, characterised in that at least one vertically arranged ferrite antenna is provided for receiving spherics pulses having a horizontally polarised magnetic field component, and in that a further, horizontally arranged antenna (5) is provided for receiving spherics pulses having a vertically polarised magnetic field component, in that spherics selectors (10 to 13, 17) are connected after the antennas, said spherics selectors (10 to 13, 17) comprising filters (35, 36) for the respective frequencies and circuit means (37) acting as an interlock in the case of permanent technical vibrations, and—in the case of the spherics selectors (10 to 13) for the spherics pulses having a vertically polarised magnetic field—further logical circuit means (38, 39, 45) for blocking off any EMP pulses occurring, and in that further logical circuit units (60) will then pass the pulse rates of the respective spherics pulses to a subtracting network (62) once they exceed a pre-determined value, and in that in the subtracting network, the pulse rates of the 10 kHz spherics pulses are subtracted from those of the 28 kHz spherics pulses, and in that the output signal of this subtracting network (62) represents the pre-warning signal, and in that a further network (63) is provided, which is reached by the output signal from said subtracting network (62), and which is also reached by the 50 kHz spherics pulses, and which will only pass the output signals from the former subtracting network (62), if no 50 kHz spherics pulses are present.

4. Circuit arrangement according to Claim 3, characterised in that the frequency of occurrence of the spherics pulses, after which said circuit means (60) will pass the number of the spherics pulses measured by the spherics selectors per time unit to the subtracting network, is 1.0 Hz.

5. Circuit arrangement according to Claim 3, characterised in that for limiting the range of the antennas the pre-amplifier connected in front of the spherics selectors (10 to 13, 15 to 20) is controlled by means of a control signal which depends on the intensity of the signal received by means of a further antenna (21) from a control transmitter transmitting at a constant field strength.

Fig.1

EP 0 120 991 B1

Fig.2

Fig.3

Sphärik-Selektor

Fig.4

2

Fig.5

$$\Delta F = F (28) - F (10)$$

Januar 1981

Fig.6